## Europäisches Patentamt

## European Patent Office

## Office européen des brevets

(11) Veröffentlichungsnummer: **0 002 777**
**A1**

(12) **EUROPÄISCHE PATENTANMELDUNG**

(21) Anmeldenummer: **78101753.8**

(22) Anmeldetag: **19.12.78**

(51) Int. Cl.²: **A 61 K 9/00**

(30) Priorität: **29.12.77 DE 2758578**

(43) Veröffentlichungstag der Anmeldung: **11.07.79**
**Patentblatt 79/14**

(84) Benannte Vertragsstaaten: **BE CH DE FR GB IT NL SE**

(71) Anmelder: **HOECHST AKTIENGESELLSCHAFT,**
**Zentrale Patentabteilung Postfach 80 03 20, D-6230**
**Frankfurt/Main 80 (DE)**

(72) Erfinder: **König, Wolfgang, Dr., Eppsteiner Strasse 25,**
**D-6238 Hofheim am Taunus (DE)**
Erfinder: **Leeb, Richard, Dr., Nonnbornstrasse 15,**
**D-6233 Kelkheim (Taunus) (DE)**
Erfinder: **Bickel, Martin, Dr., Breitlacherstrasse 25,**
**D-6000 Frankfurt/Main 90 (DE)**

(54) **Stabile Sekretinzubereitung, Verfahren zu ihrer Herstellung und ihre Verwendung.**

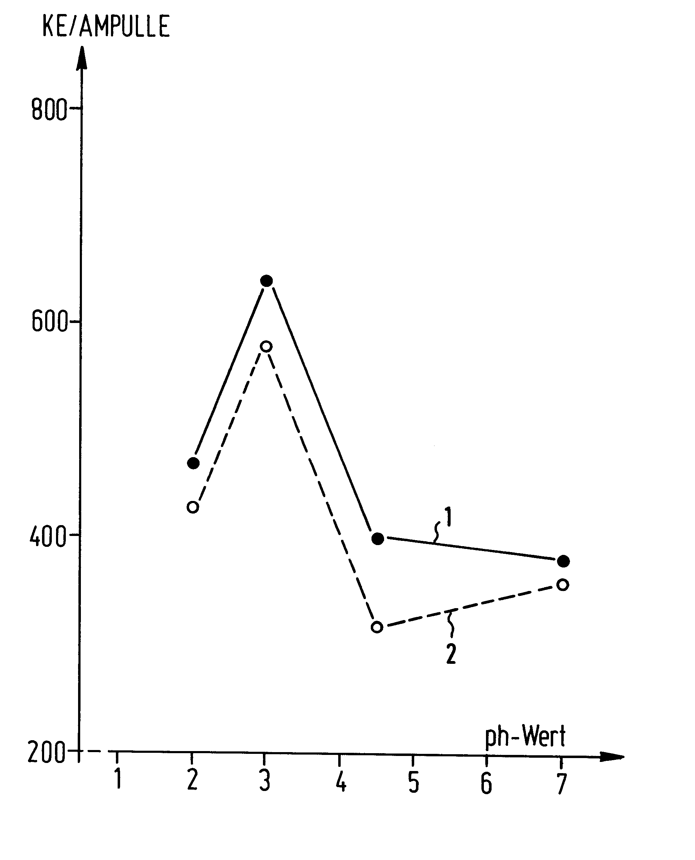

(57) Stabile Sekretinzubereitung, welche einen pH-Wert von 3,0 aufweist, Verfahren zur ihrer Herstellung und ihre Verwendung zur Behandlung von akuten Magenblutungen, Ulcus duodeni, Ulcus ventriculi, Zollinger-Ellison-Syndrom, Mucoviscidose und Reflux oesophagetis, zur Prophylaxe von Stressulcera und zur Diagnose der Pankreasfunktion und von Ulcus duodeni. Das Aktivitätsmaximum liegt bei pH 3 wie dargestellt in Figur 1.

- 1 -

HOECHST AKTIENGESELLSCHAFT HOE 77/F 263  **Dr.D/wö**

Stabile Sekretinzubereitung, Verfahren zur ihrer Herstellung
und ihre Verwendung

Die Erfindung betrifft eine stabile Sekretinzubereitung
für Injektionszwecke, Verfahren zu ihrer Herstellung und
ihre Verwendung als Arzneimittel, Prophylaktikum und
Diagnostikum.

Sekretin ist ein Heptacosipeptidamid (Eur. J.Biochem. 15,
513 (1970)), das im Duodenum gebildet wird und neben
anderen Eigenschaften vor allem die Hydrogencarbonatproduktion der Bauchspeicheldrüse stimuliert und die Wirkung
von Gastrin hemmt. Es ist deshalb zur Diagnose und zur
Therapie von Krankheiten des Verdauungstraktes verwendbar.

In den vorstehenden und nachfolgenden Ausführungen schließt
der Begriff Sekretin die physiologisch verträglichen Salze
mit ein. Erfindungsgemäß wird sowohl natürliches als auch
synthetisches Sekretin verwendet.

Es ist bekannt, daß Sekretin sehr instabil ist und vor
allem in Lösung bei Raumtemperatur schnell seine biologische Wirkung verliert (Methods in Investigative and Diagnostic Endocrinology, Vol. 2B, Seite 1067, North Holland Publishing Co., Amsterdam, London 1973). Sekretin wird deshalb in gefriergetrockneter Form gelagert und erst kurz vor Gebrauch gelöst.

Da Sekretin häufig in sehr geringen Mengen (z.B. 0,025 bis 0,2 mg entsprechend etwa 100 bis 800 KE (KE bedeutet klinische Einheit )) verabreicht wird, ist es zweckmäßig, der Sekretinlösung vor der Lyophilisierung eine Trägersubstanz zuzusetzen, um die Gefahr einer Verstaubung zu vermeiden.

Es ist aus der Literatur bekannt, daß die üblicherweise verwendeten Trägerstoffe wie Dextran und Mannit nicht günstig auf die Stabilität von Sekretin einwirken (DE-OS 2 323 187). Entsprechend den Angaben in Gastroenterology 57, 767 (1969) ist Cysteinhydrochlorid als Trägerstoff mit gutem Einfluß auf die Stabilität geeignet. Nachteilig ist jedoch, daß das so stabilisierte Sekretin nicht mehr radioaktiv jodiert werden kann und daher für die Verwendung von Radioimmunoassays ungeeignet ist. Auch eine mögliche Oxydation des leicht löslichen Cysteins zum schwerlöslichen Cystin könnte Löslichkeitsprobleme (Trübung der Lösung) mit sich bringen.

In der deutschen Offenlegungsschrift 2 323 187 werden verschiedene Aminosäuren auf ihre Verwendbarkeit als Trägersubstanzen bei Sekretinzubereitungen getestet. Dabei fand man, daß bei einem pH von 4-4,5 Alanin besser abschneidet als z.B. Glycin, Threonin oder Valin und daß daher Alanin als Trägersubstanz stabiler Sekretinzubereitungen geeignet ist.

Wir haben nun gefunden, daß die Stabilität von Sekretinzubereitungen stark abhängig ist vom pH-Wert und der Temperatur der Lösung und daß sich auf pH 3 abgepuffertes Glycin als Trägersubstanz besonders eignet.

Gegenstand der Erfindung ist daher eine stabile gefriergetrocknete Sekretinzubereitung, die mit Salzsäure auf einen
pH-Wert von 3 eingestellt wurde und gegebenenfalls auf pH 3
abgepuffertes Glycin als Trägersubstanz enthält.

Die Erfindung betrifft ferner ein Verfahren zur Herstellung
einer stabilen, gefriergetrockneten Sekretinzubereitung,
das dadurch gekennzeichnet ist, daß man einer mit Salzsäure
auf pH 3 eingestellten, auf etwa 0 bis 10°C gekühlten
wäßrigen Lösung, welche gegebenenfalls mit Salzsäure auf
pH 3 gepuffertes Glycin enthält, Sekretin zufügt, die
Lösung steril filtriert, in Flaschen oder Ampullen abfüllt
und gefriertrocknet.

In Figur 1 ist die biologische Aktivität einer Sekretinzubereitung enthaltend 0,1 mg Sekretinhydrochlorid und 20 mg
Glycin als Trägersubstanz dargestellt. Die Kurven 1 und 2
geben nicht nur Auskunft über die Aktivität in Abhängigkeit vom pH-Wert der Zubereitung, sondern auch über die
Abhängigkeit von der Temperatur. Das Präparat, welches
erst nach 68-stündigem Stehen bei Raumtemperatur gefriegetrocknet wurde (Kurve 2), hat im Vergleich zum Präparat,
welches sofort lyophilisiert wurde (Kurve 1) an Aktivität
verloren. Das Aktivitätsmaximum liegt bei pH 3.

Zur Vermeidung des Aktivitätsverlustes durch Temperatureinflüsse wird bei dem erfindungsgemäßen Verfahren die
sekretinhaltige Lösung möglichst unverzüglich lyophilisiert.

Bei geringer Sekretindosierung (0,025 mg/Ampulle) wurde
die Beobachtung gemacht, daß auch Zubereitungen bei pH 3
eine schwächere biologische Wirkung zeigten als zu erwarten war.

Es wurde jedoch gefunden, daß diese Präparate die volle
Aktivität nach Zusatz von Gelatinepartialhydrolysat, das
über Diisocyanate (verg. DBP 1 118 792 und 1 155 134) vernetzt ist($^{(R)}$Haemaccel), Human-Albumin oder Human-Globin zurückerhalten. Offensichtlich handelte es sich hier nicht um eine Desaktivierung des Sekretins, sondern um eine Adsorption des Sekretins an der Glasoberfläche der Ampulle, die durch Zusatz von Proteinen oder proteinähnlichen Substanzen verhindert bzw. rückgängig gemacht werden kann.

Zubereitungen mit geringerem Sekretingehalt erhalten daher zweckmäßigerweise die genannten Zusätze. Sie können bei hochdosierten Präparaten (z.B. 800 KE und mehr pro Ampulle) fehlen.

Um eine protrahierte Wirkung des Sekretins zu erreichen, kann die erfindungsgemäße Sekretinzubereitung vor Applikation mit einem Depotträger vermischt werden. Als Depotträger dient vorzugsweise eine Mischung von Polyphloretinphosphat und mit Diisocyanaten vernetztem Gelatinepartialhydrolysat (vergl. DBP 2 104 344).

Da die Sekretinzubereitungen injiziert werden, ist es zweckmäßig, vor Applikation das Lyophilisat in Aqua pro injectione, das einen physiologisch verträglichen Puffer enthält, zu lösen.

Die erfindungsgemäßen Sekretinzubereitungen sind sowohl als Pankreasfunktionsdiagnosticum und zur Diagnose von Ulcus duodeni als auch als Therapeutikum bei Ulcus duodeni, Ulcus ventriculi, Stressulcera, Blutungen im Gastroduodenalbereich, Refluxoesophagitis, Mucoviscidose und Zollinger-Ellison-Syndrom und zur Prophylaxe von Stressulcera z.B. nach Operationen, multiplen Frakturen, Verbrennungen und Septikämien geeignet.

Als Pankreasfunktionsdiagnosticum verwendet man bevorzugt eine 10 ml-Ampulle mit 100 KE. Der Inhalt der Ampulle wird in 10 ml physiologischer Kochsalzlösung gelöst und langsam intravenös appliziert. Auch eine Infusion ist zu diesem Zweck möglich.

Die erfindungsgemäße Sekretinzubereitung ist auch geeignet zur Diagnose von Ulcus duodeni, denn es wurde bei Ulcus-Patienten und bei Probanden, die früher ein Zwölffingerdarmgeschwür hatten, nach intracutaner Gabe von Sekretin eine positive Hautreaktion beobachtet. Diese Beobachtung wird daher zur Diagnose herangezogen. Sekretin wird hierbei in Dosierungen von vorzugsweise 5 bis 10 KE appliziert.

Blutungen im Gastroduedenalbereich werden mit Sekretininfusionen behandelt. Man gibt etwa 0,5 KE/kg Körpergewicht innerhalb einer Stunde. Die Infustionsdauer beträgt in der Regel 48 Stunden. Zu diesem Zweck ist besonders die 200 KE-Ampulle geeignet, die für etwa 4 - 5 Stunden ausreicht. Es empfiehlt sich alle 4 - 5 Stunden eine neue Lösung anzusetzen.

Bei der Therapie des Ulcus duodeni und Ulcus ventriculi verwendet man bevorzugt einen Depotträger analog DE-PS 2 104 344, der die Sekretinwirkung am Pankreas (Hydrogencarbonatsekretion) und Magen (Gastrinhemmung) mehrere Stunden aufrechterhält. Der Depotträger ist aus Stabilitätsgründen der Sekretinzubereitung nicht beigemischt, sondern wird erst unmittelbar vor Applikation mit Sekretin vermischt. Ein weiterer Grund für die getrennte Aufbewahrung von Depotträger und Sekretinzubereitung ist die Möglichkeit einer genauen Sekretindosierung, die vom Gewicht des Patienten abhängt. Die Dosis beträgt bei dieser Therapie vorzugsweise 10 KE/kg Körpergewicht. Für diese Verwendung ist z.B. eine 800 KE-Ampulle geeignet. Der Depotkörper und die erfindungsgemäße Sekretinzubereitung werden zusammen in 1 ml bidestilliertes Wasser gelöst und subcutan injiziert. Man gibt täglich 1 - 2 Injektionen dieser Depotträger/Sekretin-Mischung und kann die Therapie bis zu 6 Wochen ausdehnen. Diese Depotzubereitung kann auch zur Behandlung des Zollinger-Ellison-Syndroms der Mucoviscidose und der Refluxoesophagitis sowie zur Prophylaxe von Stressulcera wie z.B. nach Operationen, multiplen Frakturen,
Verbrennungen und Septikämien angewandt werden.

Beispiel 1: 100 KE-Ampulle

Eine auf ca. 5°C gekühlte Lösung von 20 g Glycin in 400 ml
Wasser wird mit 1N HCl auf pH 3 eingestellt. Dazu gibt
man 2,5 g mit Diisocyanaten vernetztes Gelatinepartialhydrolysat (Haemaccel$^{(R)}$) in Form einer ca. 10-prozentigen
Lösung. In diese Trägerlösung werden 100 000 KE Sekretinhydrochlorid gelöst. Mit Wasser wird auf 500 ml aufgefüllt.
Die Lösung wird durch ein geeignetes Filter steril filtriert und anschließend zu je 0,5 ml in Ampullen abgefüllt
und gefriergetrocknet.

Beispiel 2: 100 KE-Ampulle

Analog Beispiel 1. Statt 2,5 g Haemaccel gibt man 1 g
Human-Albumin zu.

Beispiel 3: 200 KE-Ampulle

Analog Beispiel 1. Statt 100 000 gibt man 200 000 KE
Sekretinhydrochlorid zu.

Beispiel 4: 200 KE-Ampulle

Analog Beispiel 3.  Statt 2,5 g Haemaccel gibt man 0,5 g
Human-Globin zu.

Beispiel 5: 800 KE-Ampulle

Analog Beispiel 1. Statt 100 000 KE gibt man 800 000 KE
Sekretinhydrochlorid zu. Die 2,5 g Heamaccel werden nicht
zugegeben.

Beispiel 6:   10 KE-Ampulle

Analog Beispiel 1. Statt 100 000 KE gibt man 10 000 KE
Sekretinhydrochlorid zu.

Beispiel 7: Herstellung des Depotträgers

3 g Polyphloretinphosphat werden unter Rühren in 1N NaOH
gelöst und die Lösung auf ein pH von 6,8 eingestellt. Zu
dieser Lösung gibt man 8 g Heamaccel als 10-pozentige
Lösung. Anschließend füllt man mit destilliertem Waser auf
200 ml auf, filtriert steril und füllt zu je 2 ml in eine
Rollrandflache und gefriertrocknet.

0002777

Patentansprüche:

1. Stabile Sekretinzubereitung, dadurch gekennzeichnet, daß sie einen pH-Wert von 3,0 aufweist, wobei dieser mit Salzsäure eingestellt wurde.

2. Zubereitung gemäß Anspruch 1, dadurch gekennzeichnet, daß sie als Trägersubstanz auf pH 3 abgepuffertes Glycin enthält.

3. Zubereitung gemäß Anspruch 2, dadurch gekennzeichnet, daß sie pro Dosierungseinheit 5 bis 50 mg auf pH 3 abgepuffertes Glycin als Träger enthält.

4. Zubereitung gemäß Ansprüchen 1 bis 3, dadurch gekennzeichnet, daß sie ein mit Diisocyanaten vernetztes Gelatinepartialhydrolysat, Human-Albumin oder Human-Globin enthält.

5. Zubereitung gemäß Ansprüchen 1 bis 4, dadurch gekennzeichnet, daß sie einen Depotträger bestehend aus Polyphloretinphosphat und einem mit Diisocyanaten vernetzten Gelatinepartialhydrolysat enthält.

6. Zubereitung gemäß Ansprüchen 1 bis 5, dadurch gekennzeichnet, daß sie Sekretinhydrochlorid, Sekretinhydrobromid oder ein Gemisch dieser Salze enthält.

7. Verfahren zur Herstellung einer Zubereitung gemäß Anspruch 1, dadurch gekennzeichnet, daß man einer mit Salzsäure auf pH 3 eingestellten und auf etwa 0 bis 10°C gekühlten wäßrigen Lösung Sekretin zufügt, die Lösung steril filtriert, in Flaschen oder Ampullen abfüllt und gefriertrocknet.

8. Verfahren gemäß Anspruch 7, dadurch gekennzeichnet, daß die auf pH 3 eingestellte wäßrige Lösung mit Salzsäure auf pH 3 gepuffertes Glycin enthält.

0002777

9. Verfahren gemäß Ansprüchen 7 und 8, dadurch gekennzeichnet, daß man der wäßrigen Lösung ein mit Diisocyanaten vernetztes Gelatinepartialhydrolysat, Human-Albumin oder Human-Globin zusetzt.

10. Verfahren gemäß Ansprüchen 7 bis 9, dadurch gekennzeichnet, daß man unmittelbar vor Applikation der Zubereitung Aqua pro injectione oder eine physiologisch verträgliche Pufferlösung zusetzt.

11. Verfahren gemäß Ansprüchen 7 bis 10, dadurch gekennzeichnet, daß man unmittelbar vor Applikation der Zubereitung einen Depotträger bestehend aus einem mit Diisocyanaten vernetzten Gelatinepartialhydrolysat und Polyphloretinphosphat zusetzt.

12. Verwendung einer Sekretinzubereitung gemäß Ansprüchen 1 bis 6 bei der Behandlung von akuten Magenblutungen, Ulcus duodeni, Ulcus ventriculi, Zollinger-Ellison-Syndrom, Mucoviscidose und Reflux oesophagetis, zur Prophylaxe von Stressulcera und zur Diagnose der Pankreasfunktion und von Ulcus duodeni.

0002777

# 0002777

Nummer der Anmeldung

## Europäisches Patentamt

## EUROPÄISCHER RECHERCHENBERICHT

EP 78 10 1753

| Kategorie | Kennzeichnung des Dokuments mit Angabe, soweit erforderlich, der maßgeblichen Teile | betrifft Anspruch |
|---|---|---|
| X | FR - A - 2 347 337 (HOECHST)  * Seite 1, Zeile 35 - Seite 2, Zeile 13; Seite 9, Zeilen 1-7 * | 1-3, 6-8, 12 |
| | --- | |
| | FR - A - 2 123 547 (HOECHST)  * Seite 1, Zeile 1 - Seite 2, Zeile 5; Seite 5, Zeilen 1-33 * | 1,4,5, 9-12 |
| | --- | |
| A | DE - A - 2 107 282 (PAULSEN) | 1 |
| | ---- | |

**KLASSIFIKATION DER ANMELDUNG (Int.Cl.²)**

A 61 K 9/00

**RECHERCHIERTE SACHGEBIETE (Int. Cl.²)**

A 61 K 9/00
C 07 C 103/

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 02-04-1979 | JONAS |

EPA form 1503.1 06.78